# EUROPEAN PATENT SPECIFICATION

(11) **EP 3 135 195 B1**
(45) Date of publication and mention of the grant of the patent: **20.09.2023**
(21) Application number: 16188056.2
(22) Date of filing: 12.03.2014
(51) Int. Cl.: G16H 40/63, H04W 4/38, A61B 5/145

(54) **METHOD FOR TRANSMITTING A GLUCOSE MEASURE AND HANDHELD GLUCOSE METER**
VERFAHREN ZUR ÜBERTRAGUNG EINER GLUCOSEMESSUNG UND TRAGBARES GLUCOSEMESSGERÄT
PROCEDE DE TRANSMISSION D'UNE MESURE DE GLUCOSE ET GLUCOMETRE MANUEL

(30) Priority: 12.03.2013 US 201313794919; 12.03.2013 US 201313794985
(43) Date of publication of application: 01.03.2017
(62) Divisional of application: 14709942.8
(73) Proprietor: Roche Diabetes Care GmbH, 68305 Mannheim (DE); F. Hoffmann-La Roche AG, 4070 Basel (CH)
(72) Inventor: Batman, Carol J., Indianapolis, IN 46254 (US); Carlson, Craig L., Fishers, IN 46037 (US); Celentano, Michael J., Fishers, IN 46037 (US); Davis, Stacia, Indianapolis, IN 46236 (US); Markisohn, David Bradley, Indianapolis, IN 46256 (US); McKinney, Erin K., Brownsburg, IN 46112 (US); Mears, Mark G., Westfield, IN 46074 (US); Stanley, Amy S., Noblesville, IN 46060 (US); Stickrod, Benjamin E., Sherwood, OR 97140 (US)
(74) Representative: Bittner, Thomas L.

(56) References cited:
- WO-A2-2012/067854
- JP-A- 2003 032 626
- JP-A- 2004 199 224
- US-A1- 2002 019 707
- US-A1- 2003 134 625
- US-A1- 2008 270 186
- US-A1- 2008 284 855
- US-A1- 2010 142 692
- US-A1- 2010 279 418
- US-A1- 2010 331 654
- US-A1- 2011 028 096
- US-A1- 2011 205 064
- US-A1- 2012 130 646
- US-A1- 2012 245 447
- DEFENSE ADVANCED RESEARCH PROJECTS AGENCY INFORMATION SCIENCES INSTITUTE UNIVERSITY OF SOUTHERN CALIFORNIA 4676 ADMIRALTY WAY MARI: "INTERNET PROTOCOL DARPA INTERNET PROGRAM PROTOCOL SPECIFICATION ; rfc791.txt", NETWORK WORKING GROUP RFC 1717, INTERNET SOCIETY (ISOC) 4, RUE DES FALAISES CH- 1205 GENEVA, SWITZERLAND, CH, 1 September 1981 (1981-09-01), XP015006773, ISSN: 0000-0003

## Description

The present disclosure relates to techniques for transferring data from a handheld glucose meter automatically to a portable communication device.

### Background

Persons with diabetes have difficulty regulating blood glucose levels in their bodies. As a consequence, many of these persons carry specialized electronic meters, called blood glucose meters, which allow them to periodically measure their glucose levels and take appropriate action, such as administering insulin. These persons may also carry with them a portable communication device, such as a mobile phone, a personal digital assistant, a tablet or similar device. People often rely on their portable communication device as the primary means for planning, scheduling and communicating with others. As a result, most portable communication devices are equipped with sophisticated software which provides user-friendly means for viewing and inputting data. Accordingly, a person with diabetes may wish to wirelessly transmit the results of a blood glucose measurement from their glucose meter to their portable communication device in order, for example, to display, analyze or report on the data.

Therefore, it is desirable to develop improved techniques for transferring blood glucose measures from a glucose meter automatically via a wireless data link to a diabetes management application residing on a portable communication device.

Document US 2008/0284855 A1 discloses an electronic camera having a wireless communication unit that performs a wireless communication with a computer or a printer.

Document US 2012/0245447 A1 describes analyte monitoring systems, devices, and methods associated with analyte monitoring devices, and devices incorporating the same. Various graphical user interfaces (GUI) and navigation flows are provided for performing various features, activities, functions, etc., associated with the analyte monitoring device or system. Intuitive navigation is provided to enhance the interpretation of analyte measurements.

Document US 2008/0270186 A1 relates to a method comprising the steps of: subscribing with an identification code of a drug administration device to a health care advisory system, transferring the identification code of the drug administration device to the health care advisory system, controlling that the patient having the drug administration device is a subscriber to the health care advisory system based on the received identification code, requesting and receiving treatment data from the subscribing drug administration device, generating an advice based on the received treatment data and a personalized setup of patient related data in the health care advisory system, and/or generating an order based on the received treatment data in the health care advisory system to supply goods to the patient, and sending the advice to the drug administration device, and/or sending the order to a goods supplier, said method further comprises the step of charging the patient having the drug administration device for the received service.

Document US 2002/0019707 A1 discloses a glucose meter that includes means for processing a blood sample to provide a glucose measurement and a communication interface to send and receive data including the glucose measurement to a remote processing center. The processing center collects and processes a plurality of glucose measurements to provide a modified method for processing a blood sample in order to provide an adjusted glucose measurement. In one embodiment, the modified blood sample processing method is a function of a permissible glucose deviation for the patient and in another embodiment, the modified blood sample processing method is a function an aging factor indicative of test strip variations overtime.

In Document US 2010/0142692 A1, a system for performing a teleassistance service, for example, in the medical field, is disclosed. The system comprises a central system for controlling and managing the service, a local system for performing the service, and a telecommunication network adapted to communicate the central system with the local system, wherein the local system includes at least one sensor adapted to perform measures of at least one biomedical parameter on a user and a terminal adapted to communicate with the sensor for receiving information related to measure results and adapted to transmit said information to the central system through the telecommunications network, and wherein at least one between the terminal and the central system includes a software agent provided with a process engine for executing workflows, said agent being adapted to interact with the sensor when executing workflows.

Document WO 2012/067854 A2 discloses systems and methods to utilize factual information based on stored analyte data to allow greater insight into the management of diabetes of a user. Details of the Internet Protocol are disclosed in document XP015006773 (Defense Advanced Research Projects Agency / Information Sciences Institute, University of Southern California, Internet Protocol DARPA Internet Program Protocol Specification; rfc791.txt, 1 September 1981, ISSN 0000-0003).

Document US2010331654 A1 discloses a method and device for a glucose meter having a user interface for displaying status during transmission of a blood glucose measure.

### Summary

A computer-implemented method for transmitting a glucose measure according to claim 1 and a handheld glucose meter according to claim 11 are provided. Further embodiments are subject matter of dependent claims.

Persons with diabetes often carry a handheld glucose meter as well as a portable computing device, such as a mobile phone. Given the close proximity of these two devices, the portable computing device can serve as a data collector for the glucose measures taken by the glucose meter. Improved techniques are set forth for transferring glucose measures automatically and seamlessly to the patient's portable computing device.

In another aspect, a handheld glucose meter having a user interface for displaying status during transmission of a blood glucose measure is provided, comprising: a port configured to receive a test strip having a reaction site for receiving a sample of blood from a patient; a glucose measurement module cooperatively operable with a test strip inserted in the port to measure glucose in a sample of blood residing on the test strip: a user interface module configured to receive the glucose measure from the glucose measurement module and tag the glucose measure with a unique sequence number form a counter, the user interface module further operates to display the glucose measure on a result screen of the glucose meter immediately following the determination of the glucose measure by the glucose measurement module; a wireless transceiver in data communication with the user interface module and operable to communicate a message automatically via a wireless data link to a diabetes management application residing on a portable computing device, where the transmission occurs automatically in response to navigating away from the result screen and the message includes only one glucose measure.

Also, a method is provided for transferring glucose measure automatically from a handheld glucose meter to a diabetes management application residing on a portable computing device. The method includes: determining a blood glucose measure from a test strip inserted into a port of the glucose meter; tagging the glucose measure with a unique sequence number, where the sequence number is determined from a counter residing on the glucose meter; displaying the measurement result screen on a display of the glucose meter, where the measurement result screen includes a numeric value for the glucose measure and is displayed in response to the determination of the blood glucose measure; and transmitting a message via a wireless data link to the diabetes management application, where transmission occurs automatically and the message includes only one glucose measure.

Further, a handheld glucose meter is presented. The glucose meter includes: a port configured to receive a test strip having a reaction site for receiving a sample of blood from a patient; a glucose measurement module cooperatively operable with a test strip inserted in the port to measure glucose in a sample of blood residing on the test strip; and a user interface module configured to receive the glucose measure from the glucose measurement module and tag the glucose measure with a unique sequence number from a counter. The user interface module further operates to display the glucose measure on a result screen of the glucose meter immediately following the determination of the glucose measure by the glucose measurement module. The glucose meter further includes a wireless transceiver in data communication with the user interface module. The wireless transceiver communicates a message automatically via a wireless data link to a diabetes management application residing on a portable computing device, where the transmission occurs automatically in response to navigating away from the result screen and the message includes only one glucose measure.

In another embodiment, a computer-implemented method for transmitting a glucose measure automatically from a handheld glucose meter to a diabetes management application residing on a portable computing device is provided. The method comprising steps of determining, by the glucose meter, a blood glucose measure from a test strip inserted into a port of the glucose meter, the test strip having a reaction site for receiving a sample of blood from a patient; tagging, by the glucose meter, the glucose measure with a unique sequence number, where the sequence number is determined from a counter residing on the glucose meter; displaying, by the glucose meter, the measurement result interface on a display of the glucose meter, where the measurement result interface includes a numeric value for the glucose measure and is displayed in response to the determination of the blood glucose measure; transmitting, by the glucose meter, a message via a wireless data link to the diabetes management application, where transmission occurs automatically in response to navigating away from the measurement result interface and the message includes only one glucose measure; receiving, by the glucose meter, a request for a missing glucose measure, where the request identifies the missing glucose measure by a sequence number assigned by the glucose meter and the request is sent via the wireless data link by the diabetes management application in response to receiving the message; and transmitting, by the glucose meter, the missing glucose measure via the wireless data link to the diabetes management application, where the transmission occurs automatically in response to receiving the request.

In yet another embodiment, a computer-implemented method for transmitting a glucose measure automatically from a handheld glucose meter to a diabetes management application residing on a portable computing device is disclosed. The method is comprising: determining, by the glucose meter, a blood glucose measure from a test strip inserted into a port of the glucose meter, the test strip having a reaction site for receiving a sample of blood from a patient; tagging, by the glucose meter, the glucose measure with a unique sequence number, where the sequence number is determined from a counter residing on the glucose meter; incrementing, by the glucose meter, the counter by one after the step of tagging the glucose measure; displaying, by the glucose meter, the measurement result interface on a display of the glucose meter, where the measurement result interface includes a numeric value for the glucose measure and is displayed in response to the determination of the blood glucose measure; transmitting, by the glucose meter, a message via a wireless data link to the diabetes management application, where transmission occurs automatically in response to navigating away from the measurement result interface and the message includes only one glucose measure; receiving, by the glucose meter, current time from the portable computing device during the transmission of the message; synchronizing, by the glucose meter, a clock maintained by the glucose meter with the current time received from the portable computing device when a difference between time of the clock and the current time exceeds a variance threshold; receiving, by the glucose meter, a request for a missing glucose measure, where the request identifies the missing glucose measure by a sequence number assigned by the glucose meter and the request is sent via the wireless data link by the diabetes management application in response to receiving the message; and transmitting, by the glucose meter, the missing glucose measure via the wireless data link to the diabetes management application, where the transmission occurs automatically in response to receiving the request.

In the following, further embodiments are disclosed which can be applied to the methods and / or devices described herein.

The method may further comprise transferring the blood glucose measure automatically in response to the determination of the blood glucose measure and without user intervention. The method may further comprise: navigating, in response to an input command, from the result screen to a comment selection screen, the comment selection screen presenting a listing of comments for selection; receiving a selection of a comment from the listing of comments; navigating from the comment selection screen to the result screen, the navigating back to the result screen in response to receiving the selection; and transferring the blood glucose measure in response to navigating away from the result screen. The method may further comprise: pairing, by the glucose meter, with the portable computing device to thereby establish the wireless data link; prompting, by the glucose meter, input of a name for the portable computing device; and receiving, by the glucose meter, a name for the portable computing device in response to the prompt, where the name of the portable computing device serves as the identifier for the portable computing device on the interface. The method may further comprise receiving, by the glucose meter, current time from the portable computing device during the transmission of the blood glucose measure; and synchronizing, by the glucose meter, a clock maintained by the glucose meter with the current time received from the portable computing device when a difference between time of the clock and the current time exceeds a variance threshold.

The method may further comprise receiving, by the glucose meter, a request for a missing glucose measure, where the request identifies the missing glucose measure by a sequence number assigned by the glucose meter; and transmitting, by the glucose meter, the missing glucose measure via the wireless data link to the diabetes management application, where the transmission occurs automatically in response to receiving the request.

The method may further comprise transferring the blood glucose measure in accordance with a low energy feature of Bluetooth wireless technology standard.

The method may further comprise displaying, by the glucose meter, an interface on the display of the glucose meter concurrently with the transmission of the message to the diabetes management application, where the interface provides an indication of the data transfer, a numeric value for the blood glucose measure and an identifier for the portable computing device.

The method may further comprise pairing, by the glucose meter, with the portable computing device to thereby establish the wireless data link; prompting, by the glucose meter, selection of a name for the portable computing device; and receiving, by the glucose meter, a name for the portable computing device in response to the prompt, where the name of the portable computing device serves as the identifier for the portable computing device on the interface.

The method may further comprise receiving, by the glucose meter, current time from the portable computing device during the transmission of the message; and synchronizing, by the glucose meter, a clock maintained by the glucose meter with the current time received from the portable computing device when a difference between time of the clock and the current time exceeds a variance threshold.

The method may further comprise transmitting the message automatically in response to navigating from the result screen to a menu screen.

The method may further comprise retrieving the missing glucose measure from a data store using the sequence number in the request, where the retrieval is in response to receiving the request for the missing glucose measure.

The method may further comprise incrementing the counter by one after the step of tagging the glucose measure with a sequence number. The method may further comprise transmitting the message in accordance with a low energy feature of Bluetooth wireless technology standard.

The method may further comprise transmitting the message automatically in response to one of navigating away from the measurement result interface or expiration of a timeout period. The method may further comprise: receiving, by the glucose meter, a request for a missing glucose measure, where the request identifies the missing glucose measure by a sequence number assigned by the glucose meter and the request is sent via the wireless data link by the diabetes management application in response to receiving the message; and transmitting, by the glucose meter, the missing glucose measure via the wireless data link to the diabetes management application, where the transmission occurs automatically in response to receiving the request.

The wireless transceiver may transfer the glucose measure automatically in response the determination of the glucose measure by the glucose measurement module and without user intervention. The wireless transceiver may transfer the glucose measure in response to navigating away from the result screen. The user interface module may navigate, in response to an input command, from the result screen to a comment selection screen, the comment selection screen presents a listing of comments for selection; and may navigate back from the comment selection screen back to the result screen in response to receiving a comment selection; wherein the wireless transceiver transfers the blood glucose measure in response to navigating away from the result screen. The user interface module may prompt an input of a name for the portable computing device during pairing of the glucose meter with the portable computing device and receive a name for the portable computing device in response to the prompt, where the received name is displayed as the identifier of the portable computing device on the interface. The user interface module may be configured to receive current time from the portable computing device during transmission of the blood glucose measure and may operate to synchronize a clock maintained by the glucose meter with the current time received from the portable computing device when a difference between time of the clock and the current time exceeds a variance threshold. The user interface module may be configured to receive a request for a missing glucose measure from the portable computing device and may interact with the wireless transceiver to transmit the missing glucose measure via the wireless data link to the diabetes management application, where the request identifies the missing glucose measure by a sequence number assigned by the glucose meter and the transmission occurs automatically in response to receiving the request. The user interface module may operate to display an interface on the display of the glucose meter concurrently with the transfer of the glucose measure to the diabetes management application, where the interface provides an indication of the data transfer, a numeric value for the glucose measure and an identifier for the portable computing device.

This section provides a general summary of the disclosure, and is not a comprehensive disclosure of its full scope or all of its features. Further areas of applicability will become apparent from the description provided herein.

### Description of exemplary embodiments

In the following, exemplary embodiments are described with reference to figures of a drawing. The figures show:
- Figure 1: is a diagram depicting a handheld glucose meter in data communication with a diabetes management application residing on a mobile phone;
- Figure 2: is a block diagram of an exemplary hardware arrangement for the glucose meter;
- Figure 3: is a sequence diagram illustrating an exemplary sequence for taking a blood glucose measure using the glucose meter;
- Figure 4: is a flowchart illustrating an exemplary technique for transmitting blood glucose measures individually from the glucose meter;
- Figure 5: is a sequence diagram depicting an exemplary data transmission between the glucose meter and the mobile phone;
- Figure 6: depicts example screens displayed on the glucose meter during a testing scenario; and
- Figure 7: is a flowchart illustrating an exemplary technique for processing glucose measures received by the diabetes management application;

The scope of the invention is defined by the appended claims.

Corresponding reference numerals indicate corresponding parts throughout the several views of the drawings.

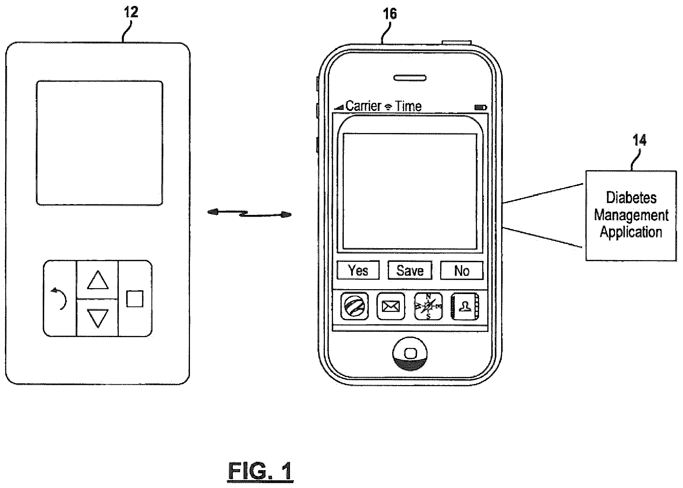

Figure 1 depicts an exemplary handheld glucose meter 12 in data communication via a wireless data link with a diabetes management application 14. The glucose meter 12 is configured to receive a sample of blood from a patient and determine a blood glucose measure for the patient from the blood sample. One or more blood glucose measures may in turn be transmitted over the wireless data link to the diabetes management application 14 for further processing. In an exemplary embodiment, the diabetes management application 14 resides on a mobile phone 16. In other embodiments, the diabetes management application may be native to a remote server with its user interface presented on the mobile phone 16. In some embodiments, data is transferred to and from the glucose meter 12 using the Bluetooth wireless technology standard (e.g., low energy feature of Bluetooth 4.0) although other types of communication transports are contemplated by this disclosure.

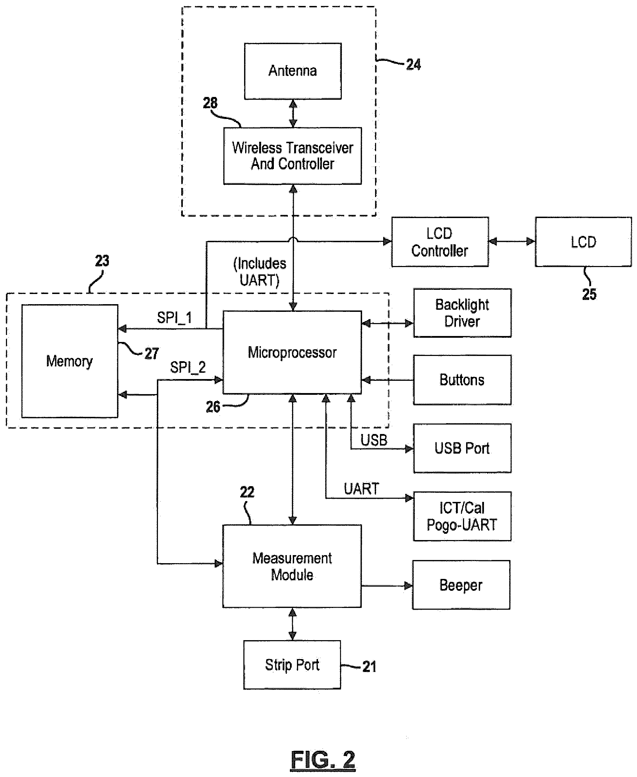

Figure 2 depicts an exemplary hardware arrangement for the glucose meter 12. The glucose meter 12 is comprised generally of a measurement module 22, a processing subsystem 23 and a communication subsystem 24. Each of these components is further described below. While the primary components are discussed herein, it is understood that other components (e.g., batteries) may be needed for the overall operational of the meter.

The measurement module 22 cooperatively interacts with a test strip inserted into a strip port 21 to determine a glucose measure from the sample of blood on the test strip. The measurement module 22 may include calibration information for the test strips being read by the meter. The calibration information may be included in a code key which can be included by the measurement module 22. As used herein, the term module may refer to, be part of, or include an application Specific Integrated Circuit (ASIC); an electronic circuit; a combinational logic circuit; a field programmable gate array (FPGA); a processor (shared, dedicated, or group) that executes code; other suitable components that provide the described functionality; or a combination of some or all of the above. The term module may further include memory that stores code executed by the processor, where code, as used above, may include software, firmware, and/or microcode, and may refer to programs, routines, functions, classes, and/or objects.

The processing subsystem 23 is configured to receive the glucose measures from the measurement module 22 which may in turn be stored (e.g. in memory) by the processing subsystem 23. Glucose measures may also be displayed by the processing subsystem 23 on a display 25. The user can interact with the meter using various user interface components, such as buttons, switches, a speaker, a microphone, USB port, etc. Each of these components is interfaced with the processing subsystem 23. In an exemplary embodiment, the processing subsystem 23 includes a microprocessor 26 and one or more volatile and/or non-volatile memories 27 although other implementations are envisioned for the processing subsystem.

The processing subsystem 23 is also interfaced with the communication subsystem 24. In an exemplary embodiment, the communication module includes a wireless transceiver 28. The wireless transceiver operates to communicate the glucose measures and other data wirelessly via a data link to a remote device physically separated from the meter. The communication subsystem can also include an antenna, microcontroller, voltage and power control circuits and a flash memory device. Although a few primary components of the meter 12 are discussed herein, it is readily understood that other components (e.g., power source) may be needed to implement the meter.

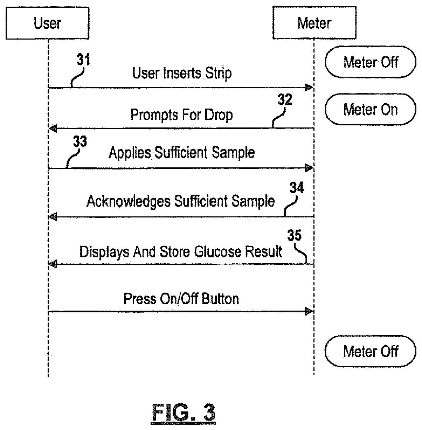

Figure 3 depicts an exemplary sequence for taking a blood glucose measure using the blood glucose meter 12. The user may insert a test strip at 31 into a port of the glucose meter. Insertion of the test strip prompts the glucose meter to power on. The user may alternatively power on the glucose meter using an on/off button. In this case, the glucose meter will prompt the user to insert a test strip. The user may also power on the glucose meter without having inserted a test strip into the meter. In any of these cases, the glucose meter may perform a quality check on the test strip inserted into the meter. Once the quality check has been completed, the meter is ready to perform a test.

To begin a test, the user is prompted at 32 for a sample of blood. In response to the prompt, the user provides a blood sample at 33 using the test strip, where the test strip includes a reaction site that receives the blood sample from the patient. Upon receipt of the blood sample, the glucose meter will proceed to analyze the blood sample in a manner readily known in the art. Before doing so, the glucose meter may acknowledge the sufficiency of the blood as indicated at 34. During the analysis, a blood glucose measure is obtained from the blood sample. The blood glucose measure will be displayed to the user and stored on the glucose meter as indicated at 35. Stored glucose measures may be uploaded subsequently from the glucose meter in a batch manner to a physician's computer.

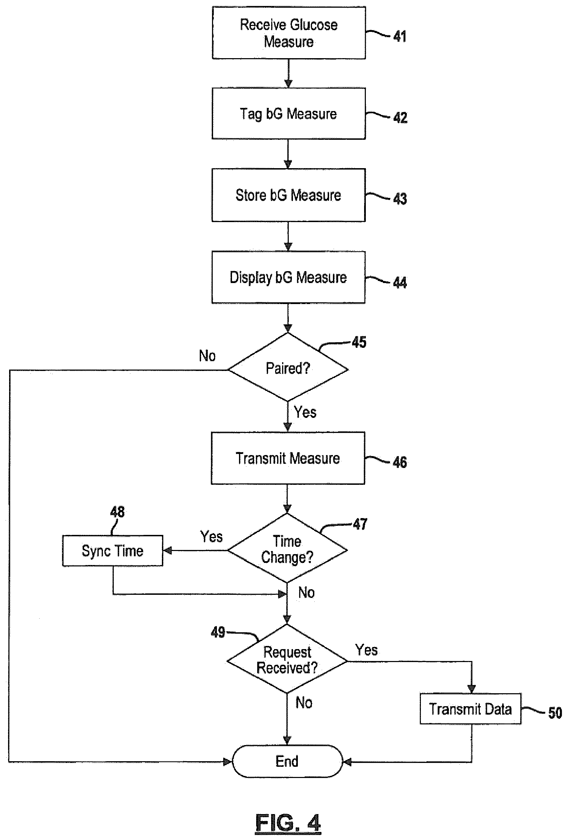

Rather than sending blood glucose measures in a batch manner, the glucose meter may be configured to transmit blood glucose measures individually as shown in Figure 4. The blood glucose measures may be transmitted, for example to a mobile phone or some other portable computing device carried by the user. Because the mobile phone is typically in close proximity to the user, it may be used as a data collector for the patient's blood glucose measures. A diabetes management application 14 residing on the mobile phone 16 can then be used for data analysis as well as other sophisticated diabetes management functions. Consequently, the processing power and memory available on the glucose meter can be streamlined, thereby reducing the cost of the glucose meter 12.

Upon determining a blood glucose measure, the blood glucose measure is first tagged at 42 with identifying information. Identifying information may include but is not limited to a name of the patient to which the measure pertains, a timestamp for when the measure was taken, a serial number for the meter and other information pertaining to the test strip. Of note, each blood glucose measure is also tagged with a unique sequence number assigned by the glucose meter. In one embodiment, a counter is incremented each time a glucose measure is taken and the value of the counter is assigned to the blood glucose measure. The sequence number may be used to retrieve missing data from the glucose meter as is further described below. Once tagged, the blood glucose measure is stored at 43 in a memory of the glucose meter and displayed to the user at 44 on a display of the glucose meter.

Next, the glucose meter determines at 45 whether it is paired via a wireless data link with another device, such as mobile phone 16. The current blood glucose measure is transmitted at 46 to the mobile phone when the glucose meter is paired to the mobile phone. While reference is made throughout this disclosure to a message being sent with a single glucose measure, it is envisioned that in some embodiments the message transmitted by the glucose meter can contain one or more glucose measures.

In one embodiment, the blood glucose measure is transmitted automatically and without user intervention. For example, after taking a glucose measure, the glucose measure is transmitted automatically after a predefined timeout period (e.g., five seconds) without receiving any input from the user. In another embodiment, the blood glucose measure is transmitted automatically in response to the user navigating away from the measurement result screen as will be further described below. In a similar manner, the blood glucose measure may be transmitted automatically in response to the meter being powered down by the user. It is envisioned that the mobile phone and/or the diabetes management application is authenticated with the glucose meter during the pairing process.

In some embodiment, the glucose meter may send the current time along with glucose measure to the mobile phone. The current time is maintained on the glucose meter by a clock residing therein. The diabetes management application may use the time from the meter when processing the glucose measure. For example, the diabetes management application may accept the glucose measure when the time falls within a certain allowable range, for example from the current time maintained by the mobile phone. Glucose measures having an associated time that falls outside an allowable range may be stored by the diabetes management application using the current time maintained by the mobile phone. Other uses for the time sent by the glucose meter are also envisioned by this disclosure.

In addition to transmitting the blood glucose measure, the glucose meter can synchronize its time with the mobile phone. During initial setup or thereafter, the glucose meter may be configured by the user, using either the blood glucose meter or the mobile phone, to synchronize its clock with the mobile phone. By enabling this time synchronization feature, the user is designating the mobile phone as the master device. Current time on the mobile phone is transmitted to the glucose meter during each data exchange. Because a user is interacting frequently with their mobile phone, the time reported by the mobile phone is likely to be accurate. The glucose meter will compare the current time on the mobile phone to the current time maintained by the glucose meter as indicated at 47. If the time synchronization feature has been enabled by the user and the difference between the two clocks exceeds a variance (e.g., 2 minutes), the glucose meter will set its clock to the current time of the mobile phone as indicated at 48. Conversely, the glucose meter may retain its current time if time synchronization feature has not been enabled or the difference between the two clocks is less than the variance threshold. In an alternative embodiment, the glucose meter will set its clock to the current time of the mobile phone if the difference between the two clocks is less than the variance threshold and the time synchronization feature is enabled. It is envisioned that other parameters, such as date/time format, target glucose ranges, hypo waning levels, etc., can also be synchronized between the two devices.

Time synchronization may occur during other types of data exchanges. For example, when the glucose meter is powered up, it may initiate a data session with the mobile phone. During this initial data session, the glucose meter may attempt to synchronize its time with the mobile phone in the manner set forth above. In this case, the time is synchronized independent from a blood glucose test.

During each data exchange, the glucose meter may also receive a request for missing glucose measures at 49 from the diabetes management application. In one embodiment, the request identifies any missing glucose measures by its sequence number as will be further described below. In response to receiving a request, the glucose meter will transmit the missing glucose measures at 50 to the diabetes management application. It is to be understood that only the relevant steps are discussed in relation to Figure 4 but that other software-implemented instructions may be needed to transmit data from the glucose meter. In an exemplary embodiment, the method described above is implemented by a user interface module residing on the glucose meter.

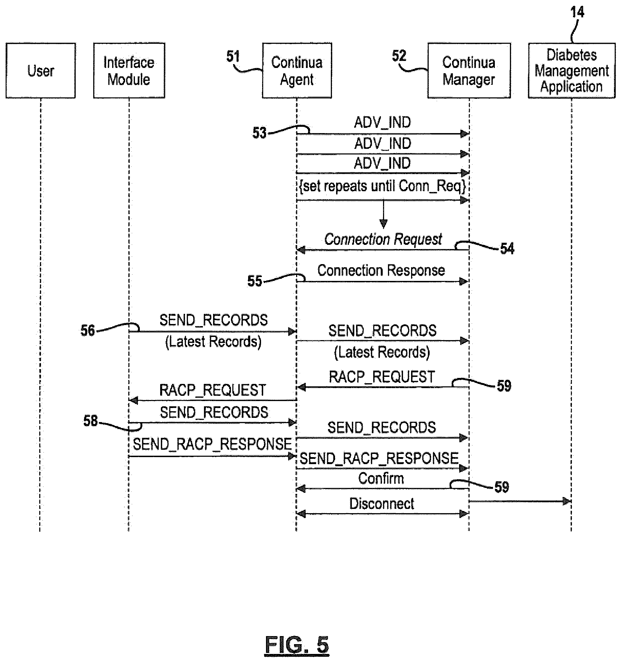

Figure 5 further illustrates an exemplary embodiment for implementing data transmission between the glucose meter 12 and the mobile phone 16. In this exemplary embodiment, data transmission can occur in accordance with any of the following specifications: IEEE standard 11073, the Bluetooth specification, and the Continua BLE specification. The communication model employs the concept of "managers" and "agents". Agents are typically smaller personal health devices that lack processing power; whereas, managers tend to be more powerful computing devices such as a mobile phone or desktop computer. Each device performs certain roles in accordance with its designation. To implement its role, each device is configured with an interface component which implements the functions associated with its designated role. In this case, the glucose meter is configured with an agent component 51 and the mobile phone is configured with a manager component 52. While reference is made to a particular communication protocol, it is readily understood that concepts disclosed herein extend more broadly to other communication protocols.

To establish a communication session, the agent 51 initiates a connection with the manager 52 as indicated at 53. In response thereto, the manager 52 sends a connection request at 54 to the agent 51. In one embodiment, the connection request may include the current time maintained by the mobile phone. The current time may be used to synchronize the time on the glucose meter as described above. The agent 51 in turn responds to the connection request as indicated at 55, thereby establishing a connection between the agent and the manager.

Once a connection has been established, the current glucose measure can be sent automatically (or "pushed") from the glucose meter 12 to the mobile phone 16. Specifically, an interface module send the glucose measure to the agent 51 which in turn transmits the glucose measure to the manager 52 as indicated at 56, where the interface module is implemented by the processing subsystem 23. In some transport mechanisms (e.g., low energy feature of Bluetooth), the data manager or collector requests data (or "pulls") from the glucose meter. The current glucose measure can be sent automatically by the glucose meter to the diabetes management application 14 before any such request is received by the glucose meter as noted above. Alternatively, the current glucose meter can be sent by the glucose meter in response to receiving the request. In an alternative embodiment, once a connection has been established, the current glucose measure can be sent from an interface module to the agent 51 which in turn transmits the glucose measure to the manager 52 as indicated at 56, where the interface module is implemented by the processing subsystem 23. The glucose measures can then be passed along the diabetes management application 14 for subsequent processing as described below.

In addition, the manager 52 may send a request at 57 for any missing glucose measures to the glucose meter. The request is relayed by the agent 51 to the interface module which in turn handles the request. That is, the interface module retrieves any missing glucose measures identified in the request and sends those glucose measures back to the agent 51 as indicated at 58. The agent 51 then sends the missing glucose measures to the manager 52. The manager 52 may confirm receipt of the missing glucose measures at 59 and then proceed to terminate the connection with the agent. In other embodiments, it is envisioned that the agent may terminate the connection. In the event there are no missing glucose measures, the manager 52 can terminate the connection without sending additional requests to or otherwise polling the glucose meter and thereby conserve power and other resources.

In some instances, the data transmission from the meter to the mobile phone may be unsuccessful. The meter may be configured to periodically attempt to resend the glucose measure to the mobile phone. If the user attempts to power down the meter before a successful data transmission, the meter may continue to periodically attempt to resend the glucose measure to the mobile phone. The next time the meter is powered on, the user may be presented a message that the glucose measure was successfully transmitted to the mobile phone. In the event the glucose measure has not yet been transmitted successfully, the user may be presented with a message indicating the same.

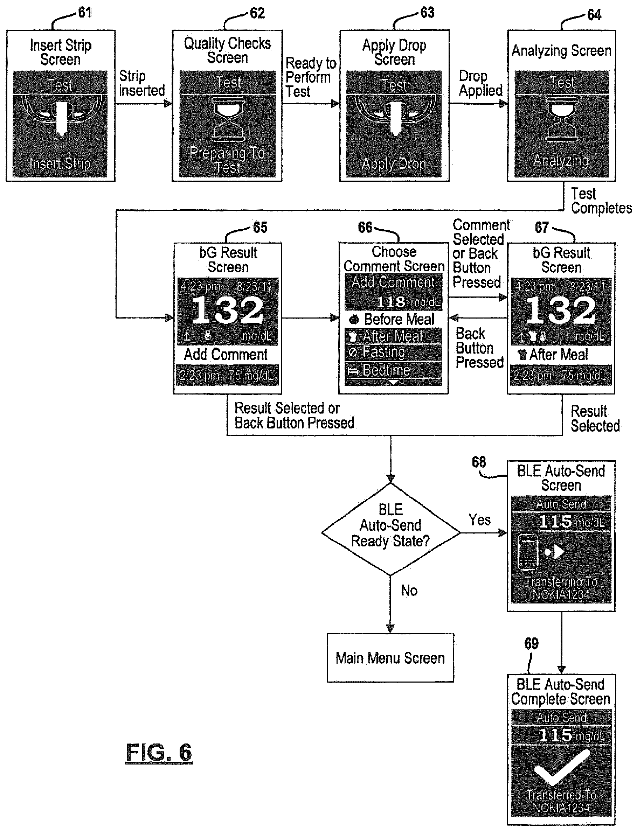

Figure 6 illustrates example screens displayed on the glucose meter during a testing scenario. From a main menu screen, the user may elect to perform a glucose test. The insert strip screen is displayed as shown at 61 when the user selects the perform test item on the main menu and a test strip is not inserted into the meter. Once a test strip has been inserted, the quality check screen appears as shown at 62 and is displayed while a quality check is performed by the meter. The quality check screen may also appear when the user selects the perform test item on the main menu and a test strip is present in the meter. Once the quality check has been completed, the meter is ready to perform a test.

To begin a test, the user is prompted to apply a blood sample as shown at 63. In response to the prompt, the user provides a blood sample using the test strip, where the test strip includes a reaction site that receives the blood sample from the patient. Upon receipt of the blood sample, the glucose meter will proceed to analyze the blood sample in a manner readily known in the art. The analyzing screen appears as shown at 64 and is displayed while the test is being performed by the meter.

Once the test completes, a blood glucose measure is displayed on the bG result screen as shown at 65. A numeric value for the blood glucose measure is displayed along with other information pertaining to the measure. Upon seeing the glucose measure, the user may elect to navigate away from the result screen, for example by depressing the <back> button on the meter. In this case, the user will return to the main menu screen and the result will be transmitted.

Alternatively, upon seeing the glucose measure, the user may elect to enter a comment pertaining to the glucose measure. To do so, the user may use the <up> or <down> buttons to select the add comment function on the screen. The choose comment screen will appear as shown at 66. In the exemplary embodiment, the user may select from a listing of comments which include before meal, after meal, fasting and bedtime. After the user selects a comment from the list, the result screen appears as shown at 67. It is noted that the selected comment is displayed along with the glucose measure on the result screen. After reviewing the annotated result, the user may elect to navigate away from the result screen.

In response to navigating way from the result screen, the glucose meter will try transmitting the glucose measure (e.g. including any comment) automatically to a paired device. The glucose meter will determine if it is paired with a device having an authenticated diabetes management application and, if so, initiate transmission of the current glucose measure to the paired device in the manner discussed above.

During data transmission, the auto-send screen is displayed as shown at 68. The auto-send screen will include an indicator that a transfer is occurring, such as a blinking arrow extending away from a meter icon. The auto-send screen will also include a value for the glucose measure being sent (i.e., 115 mg/dL) as well as an identifier for the device receiving the data transmission (i.e., NOKIA1234). Depending on the value of the glucose measure, the user may need to take some action immediately, such as administer insulin or contact a physician. Therefore, it is important that the glucose measure remain available to the user during the data transmission. Because the glucose meter may pair with many different devices, it is also important that the user be advised as to which device the glucose measure is being sent to, thereby avoiding transmission to an erroneous device. For example, only one of the available devices may be configured with an insulin recommendation function. In one embodiment, the identifier may be a serial number for the portable computing device or some other type of identifier, for example obtained from the portable computing device during the pairing process. Rather than display a serial number for the portable computing device, the glucose meter may be configured to display a more intuitive identifier for the portable computing device, such as "Tim's phone". In one embodiment, the glucose meter can prompt a user to input a name for the portable computing device, for example during or shortly after pairing with the portable computing device. The name provided by the user is stored in memory and can be displayed on the auto-send screen as described above. Upon completion of the data transfer, the auto-send complete screen appears as shown at 69.

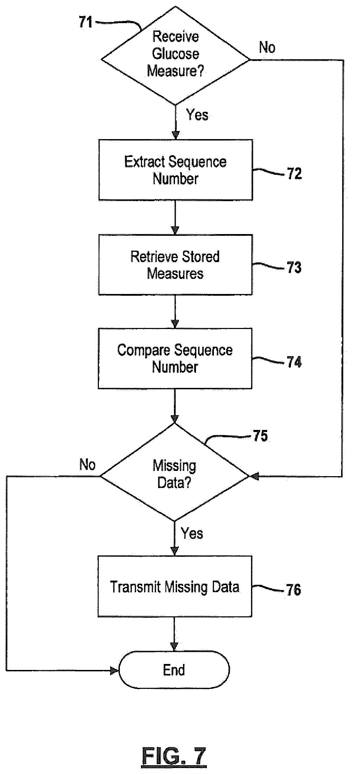

Figure 7 depicts an exemplary method for processing glucose measures received by the diabetes management application 14 residing on the mobile phone 16. In the exemplary embodiment, glucose measures are transmitted individually to the diabetes management application 14 as described in relation to Figure 4. It is envisioned that other techniques for transmitting the glucose measure to the diabetes management application 14 are contemplated by this disclosure.

Upon receiving a glucose measure, a sequence number associated with the glucose measure is first determined by the diabetes management application 14. A unique sequence number is assigned by the glucose meter to each glucose measure as described above. Thus, the sequence number associated with the glucose measure can be extracted at 72 from the data packet or message received from the glucose meter 12. In some embodiments, a series of glucose measures previously received from the glucose meter, along with their associated sequence numbers, may be stored in a memory device and thus accessible to the diabetes management application 14. In other embodiments, only the most recently received glucose measure and its sequence number is stored by the diabetes management application 14. In either case, the stored glucose measure(s) along with associated sequence number(s) are retrieved from memory.

A comparison is made at 74 between the sequence number extracted from the present glucose measure and the sequence numbers of the stored glucose measures. A request for missing glucose measures is transmitted by the diabetes management application 14 to the glucose meter 12 when an omission in the sequence is detected. For example, a request for missing glucose measures is transmitted at 76 when the extracted sequence number is 74 and the highest stored sequence number is either 71 or 72. Conversely, a request is not transmitted when the extracted sequence number is 74 and the highest stored sequence number is 73. Because this comparison is made for each glucose measure received by the diabetes management application 14, a comparison of the extracted sequence number only needs to be made to the highest stored sequence number. In other embodiments, the diabetes management application 14 may analyze the series of glucose measure for omitted measures and send a request for each glucose measure missing from the series of glucose measures. The request for missing glucose measures can be transmitted in accordance with the protocol described in relation to Figure 5. Even when a glucose measure is not received, the diabetes management application can check for omitted glucose measures as indicated at 71. As noted above, the diabetes management application can analyze the series of glucose measures for omitted measures and send a request at 76 for each glucose measure missing from the series of glucose measures. It is to be understood that only the relevant steps are discussed in relation to Figure 7 but that other software-implemented instructions may be performed by the diabetes management application 14.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The techniques described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

Some portions of the above description present the techniques described herein in terms of algorithms and symbolic representations of operations on information. These algorithmic descriptions and representations are the means used by those skilled in the data processing arts to most effectively convey the substance of their work to others skilled in the art. These operations, while described functionally or logically, are understood to be implemented by computer programs. Furthermore, it has also proven convenient at times to refer to these arrangements of operations as modules or by functional names, without loss of generality.

Unless specifically stated otherwise as apparent from the above discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices.

Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems.

The present disclosure also relates to an apparatus for performing the operations herein. This apparatus may be specially constructed for the required purposes, or it may comprise a general-purpose computer selectively activated or reconfigured by a computer program stored on a computer readable medium that can be accessed by the computer. Such a computer program may be stored in a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

## Claims

1. A computer-implemented method for transmitting a glucose measure automatically from a handheld glucose meter (12) to a diabetes management application (14) residing on a portable computing device (16), comprising:
- determining, by the glucose meter (12), a blood glucose measure from a test strip inserted into a port (21) of the glucose meter (12), the test strip having a reaction site for receiving a sample of blood from a patient;
- displaying, by the glucose meter (12), a measurement result interface on a display (25) of the glucose meter (12), where the measurement result interface includes a numeric value for the glucose measure and is displayed in response to the determination of the blood glucose measure; and
- transmitting, by the glucose meter (12), a message via a wireless data link to the diabetes management application (14);
**characterized in that**
- transmission of the message occurs automatically in response to navigating away from the measurement result interface; and
**in that** the method further comprises:
- tagging, by the glucose meter (12), the glucose measure with a unique sequence number, where the sequence number is determined from a counter residing on the glucose meter (12); and
- incrementing, by the glucose meter (12), the counter after the step of tagging the glucose measure with a sequence number.

2. The method of claim 1 further comprises transmitting the message in accordance with a low energy feature of Bluetooth wireless technology standard.

3. The method of claim 1 or 2 further comprising:
- receiving, by the glucose meter (12), a request for a missing glucose measure, where the request identifies the missing glucose measure by a sequence number assigned by the glucose meter (12) and the request is sent via the wireless data link by the diabetes management application (14) in response to receiving the message; and
- transmitting, by the glucose meter (12), the missing glucose measure via the wireless data link to the diabetes management application (14), where the transmission occurs automatically in response to receiving the request.

4. The method of one of the preceding claims further comprises displaying, by the glucose meter (12), an interface on the display (25) of the glucose meter (12) concurrently with the transmission of the message to the diabetes management application (14), where the interface provides an indication of the data transfer, a numeric value for the blood glucose measure and an identifier for the portable computing device (16).

5. The method of one of the preceding claims further comprising:
- pairing, by the glucose meter (12), with the portable computing device (16) to thereby establish the wireless data link;
- prompting, by the glucose meter (12), selection of a name for the portable computing device (16); and
- receiving, by the glucose meter (12), a name for the portable computing device (16) in response to the prompt, where the name of the portable computing device (16) serves as the identifier for the portable computing device (16) on the interface.

6. The method of one of the preceding claims further comprising:
- receiving, by the glucose meter (12), current time from the portable computing device (16) during the transmission of the message; and
- synchronizing, by the glucose meter (12), a clock maintained by the glucose meter (12) with the current time received from the portable computing device (16) when a difference between time of the clock and the current time exceeds a variance threshold.

7. The method of one of the preceding claims further comprises transmitting the message automatically in response to navigating from the measurement result interface to a menu screen.

8. The method of one of the preceding claims further comprising:
- navigating, in response to an input command, from the measurement result interface to a comment selection screen, the comment selection screen presenting a listing of comments for selection;
- receiving a selection of a comment from the listing of comments;
- navigating from the comment selection screen to the measurement result interface, the navigating back to the measurement result interface in response to receiving the selection; and
- transferring the blood glucose measure in response to navigating away from the measurement result interface.

9. The method of one of the preceding claims further comprises retrieving the missing glucose measure from a data store using the sequence number in the request, where the retrieval is in response to receiving the request for the missing glucose measure.

10. The method of one of the preceding claims wherein the counter is incremented by one after the step of tagging the glucose measure with a sequence number.

11. A handheld glucose meter (12) having a user interface for displaying status during transmission of a blood glucose measure, comprising:
- a port (21) configured to receive a test strip having a reaction site for receiving a sample of blood from a patient;
- a glucose measurement module (22) cooperatively operable with a test strip inserted in the port (21) to measure glucose in a sample of blood residing on the test strip;
- a user interface module configured to receive the glucose measure from the glucose measurement module (22), the user interface module further operates to display the glucose measure on a measurement result interface of the glucose meter (12) immediately following the determination of the glucose measure by the glucose measurement module (22);
- a wireless transceiver (28) in data communication with the user interface module and operable to communicate a message automatically via a wireless data link to a diabetes management application (14) residing on a portable computing device (16);
**characterized in that** the transmission of the message occurs automatically in response to navigating away from the measurement result interface and the message includes only one glucose measure; and
**in that** the user interface module is further configured to tag the glucose measure with a unique sequence number from a counter, and to increment the counter after the step of tagging the glucose measure with a sequence number.

12. The handheld glucose meter (12) of claim 11 wherein the user interface module is configured to receive current time from the portable computing device (16) during transmission of the blood glucose measure and operates to synchronize a clock maintained by the glucose meter (12) with the current time received from the portable computing device (16) when a difference between time of the clock and the current time exceeds a variance threshold.

13. The handheld glucose meter (12) of claim 11 or 12 wherein the user interface module navigates, in response to an input command, from the measurement result interface to a comment selection screen, the comment selection screen presents a listing of comments for selection; and navigates back from the comment selection screen back to the measurement result interface in response to receiving a comment selection; wherein the wireless transceiver (28) transfers the blood glucose measure in response to navigating away from the measurement result interface.

14. The handheld glucose meter (12) of any one of the claims 11 to 13 wherein the user interface module is configured to receive a request for a missing glucose measure from the portable computing device (16) and interacts with the wireless transceiver (28) to transmit the missing glucose measure via the wireless data link to the diabetes management application (14), where the request identifies the missing glucose measure by a sequence number assigned by the glucose meter (12) and the transmission occurs automatically in response to receiving the request.

15. The handheld glucose meter (12) of any one of claims 11 to 14 wherein the user interface module operates to display an interface on a display (25) of the glucose meter (12) concurrently with the transfer of the glucose measure to the diabetes management application (14), where the interface provides an indication of the data transfer, a numeric value for the glucose measure and an identifier for the portable computing device (16).

## Patentansprüche

1. Computerimplementiertes Verfahren zum automatischen Übertragen einer Glukosemessung von einem Glukosehandmessgerät (12) an eine Diabetesmanagementanwendung (14), die sich auf einer tragbaren Rechenvorrichtung (16) befindet, umfassend:
- Bestimmen einer Blutglukosemessung von einem Teststreifen, der in eine Öffnung (21) des Glukosemessgerätes (12) eingeführt ist, mittels des Glukosemessgerätes (12), wobei der Teststreifen über eine Reaktionsstelle zur Aufnahme einer Blutprobe von einem Patienten verfügt;
- Anzeigen einer Messergebnisschnittstelle auf einer Anzeige (25) des Glukosemessgerätes (12) mittels des Glukosemessgerätes (12), wobei die Messergebnisschnittstelle einen Zahlenwert für die Glukosemessung einschließt und in Reaktion auf die Bestimmung der Blutglukosemessung angezeigt wird; und
- Übertragen einer Nachricht über eine drahtlose Datenverbindung an die Diabetesmanagementanwendung (14) mittels des Glukosemessgerätes (12);
**dadurch gekennzeichnet, dass**
- die Übertragung der Nachricht automatisch als Reaktion auf Wegnavigieren von der Messergebnisschnittstelle stattfindet; und
dass das Verfahren ferner Folgendes umfasst:
- Markieren der Glukosemessung mit einer eindeutigen Laufnummer mittels des Glukosemessgerätes (12), wobei die Laufnummer aus einem auf dem Glukosemessgerät (12) befindlichen Zähler bestimmt wird; und
- Erhöhen des Zählers nach dem Schritt des Markierens der Glukosemessung mit einer Laufnummer mittels des Glukosemessgerätes (12).

2. Verfahren nach Anspruch 1, ferner umfassend das Übertragen der Nachricht gemäß einer Niedrigenergiefunktion des drahtlosen Bluetooth-Technologiestandards.

3. Verfahren nach Anspruch 1 oder 2, ferner umfassend:
- Empfangen einer Anfrage nach einer fehlenden Glukosemessung mittels des Glukosemessgerätes (12), wobei die Anfrage die fehlende Glukosemessung durch eine von dem Glukosemessgerät (12) zugeordnete Laufnummer identifiziert und die Anfrage über die drahtlose Datenverbindung von der Diabetesmanagementanwendung (14) als Reaktion auf das Empfangen der Nachricht gesendet wird; und
- Übertragen der fehlenden Glukosemessung über die drahtlose Datenverbindung an die Diabetesmanagementanwendung (14) mittels des Glukosemessgerätes (12), wobei die Übertragung automatisch als Reaktion auf das Empfangen der Anfrage stattfindet.

4. Verfahren nach einem der vorstehenden Ansprüche, ferner umfassend das Anzeigen einer Schnittstelle auf der Anzeige (25) des Glukosemessgerätes (12) gleichzeitig mit der Übertragung der Nachricht an die Diabetesmanagementanwendung (14) mittels des Glukosemessgerätes (12), wobei die Schnittstelle einen Hinweis auf die Datenübertragung, einen Zahlenwert für die Blutglukosemessung und eine Identifikation für die tragbare Rechenvorrichtung (16) bereitstellt.

5. Verfahren nach einem der vorstehenden Ansprüche, ferner umfassend:
- Verbinden des Glukosemessgerätes (12) mit der tragbaren Rechenvorrichtung (16), um so die drahtlose Datenverbindung herzustellen;
- Auffordern zur Auswahl eines Namens für die tragbare Rechenvorrichtung (16) mittels des Glukosemessgerätes (12); und
- Empfangen eines Namens für die tragbare Rechenvorrichtung (16) als Reaktion auf die Aufforderung mittels des Glukosemessgerätes (12), wobei der Name der tragbaren Rechenvorrichtung (16) als die Identifikation für die tragbare Rechenvorrichtung (16) an der Schnittstelle dient.

6. Verfahren nach einem der vorstehenden Ansprüche, ferner umfassend:
- Empfangen einer aktuellen Zeit von der tragbaren Rechenvorrichtung (16) während der Übertragung der Nachricht mittels des Glukosemessgerätes (12); und
- Synchronisieren einer Uhr, die von dem Glukosemessgerät (12) verwaltet wird, mit der aktuellen Zeit, die von der tragbaren Rechenvorrichtung (16) empfangen wurde, mittels des Glukosemessgerätes (12), wenn eine Differenz zwischen der Zeit der Uhr und der aktuellen Zeit eine Varianzschwelle überschreitet.

7. Verfahren nach einem der vorstehenden Ansprüche, ferner umfassend das automatische Übertragen der Nachricht als Reaktion auf das Navigieren von der Messergebnisschnittstelle zu einem Menübildschirm.

8. Verfahren nach einem der vorstehenden Ansprüche, ferner umfassend:
- Navigieren als Reaktion auf einen Eingabebefehl von der Messergebnisschnittstelle zu einem Kommentarauswahlbildschirm, wobei der Kommentarauswahlbildschirm eine Auflistung von Kommentaren zur Auswahl darstellt;
- Empfangen einer Auswahl eines Kommentars aus der Auflistung von Kommentaren;
- Navigieren von einem Kommentarauswahlbildschirm zu der Messergebnisschnittstelle, Zurücknavigieren zu der Messergebnisschnittstelle als Reaktion auf das Empfangen der Auswahl; und
- Übertragen der Blutglukosemessung als Reaktion auf das Wegnavigieren von der Messergebnisschnittstelle.

9. Verfahren nach einem der vorstehenden Ansprüche, ferner umfassend das Rückgewinnen der fehlenden Glukosemessung aus einem Datenspeicher unter Verwendung der Laufnummer in der Anfrage, wobei die Rückgewinnung als Reaktion auf das Empfangen der Anfrage für die fehlende Glukosemessung erfolgt.

10. Verfahren nach einem der vorstehenden Ansprüche, wobei der Zähler nach dem Schritt des Markierens der Glukosemessung mit einer Laufnummer um eins erhöht wird.

11. Glukosehandmessgerät (12) mit einer Benutzerschnittstelle zum Anzeigen des Status während der Übertragung einer Blutglukosemessung, umfassend:
- eine Öffnung (21), die zur Aufnahme eines Teststreifens mit einer Reaktionsstelle zur Aufnahme einer Blutprobe von einem Patienten ausgebildet ist;
- ein Glukosemessmodul (22), das zusammen mit einem in die Öffnung (21) eingeführten Teststreifen zum Messen von Glucose in einer Blutprobe, die sich auf dem Teststreifen befindet, betreibbar ist;
- ein Benutzerschnittstellenmodul, das zum Empfangen der Glukosemessung von dem Glukosemessmodul (22) ausgebildet ist, wobei das Benutzerschnittstellenmodul ferner steuert, dass die Glukosemessung unmittelbar nach der Bestimmung der Glukosemessung mittels des Glukosemessmoduls (22) an einer Messergebnisschnittstelle des Glukosemessgerätes (12) angezeigt wird;
- ein drahtloses Sende-Empfangsgerät (28) in Datenkommunikation mit dem Benutzerschnittstellenmodul, das so betrieben werden kann, dass es eine Nachricht automatisch über eine drahtlose Datenverbindung an eine Diabetesmanagementanwendung (14), die sich auf einer tragbaren Rechenvorrichtung (16) befindet, weiterleiten kann;
**dadurch gekennzeichnet, dass** die Übertragung der Nachricht automatisch als Reaktion auf Wegnavigieren von der Messergebnisschnittstelle erfolgt und die Nachricht nur eine Glukosemessung einschließt; und
dass das Benutzerschnittstellenmodul ferner dafür ausgebildet ist, die Glukosemessung mit einer eindeutigen Laufnummer von einem Zähler zu markieren und den Zähler nach dem Schritt des Markierens der Glukosemessung mit einer Laufnummer zu erhöhen.

12. Glukosehandmessgerät (12) nach Anspruch 11, wobei das Benutzerschnittstellenmodul dafür ausgebildet ist, während der Übertragung der Blutglukosemessung eine aktuelle Zeit von der tragbaren Rechenvorrichtung (16) zu empfangen und eine Uhr, die von dem Glukosemessgerät (12) verwaltet wird, mit der aktuellen Zeit, die von der tragbaren Rechenvorrichtung (16) empfangen wurde, zu synchronisieren, wenn eine Differenz zwischen der Zeit der Uhr und der aktuellen Zeit eine Varianzschwelle überschreitet.

13. Glukosehandmessgerät (12) nach Anspruch 11 oder 12, wobei das Benutzerschnittstellenmodul als Reaktion auf einen Eingabebefehl von der Messergebnisschnittstelle zu einem Kommentarauswahlbildschirm navigiert, wobei der Kommentarauswahlbildschirm eine Auflistung von Kommentaren zur Auswahl darstellt; und als Reaktion auf das Empfangen einer Kommentarauswahl von dem Kommentarauswahlbildschirm zurück zu der Messergebnisschnittstelle navigiert; wobei das drahtlose Sende-Empfangsgerät (28) die Blutglukosemessung als Reaktion auf das Wegnavigieren von der Messergebnisschnittstelle überträgt.

14. Glukosehandmessgerät (12) nach einem der Ansprüche 11 bis 13, wobei das Benutzerschnittstellenmodul dafür ausgebildet ist, eine Anfrage für eine fehlende Glukosemessung von der tragbaren Rechenvorrichtung (16) zu empfangen, und mit dem drahtlosen Sende-Empfangsgerät (28) interagiert, um die fehlende Glukosemessung über die drahtlose Datenverbindung an die Diabetesmanagementanwendung (14) zu übertragen, wobei die Anfrage die fehlende Glukosemessung durch eine von dem Glukosemessgerät (12) zugeordnete Laufnummer identifiziert und die Übertragung automatisch als Reaktion auf das Empfangen der Anfrage stattfindet.

15. Glukosehandmessgerät (12) nach einem der Ansprüche 11 bis 14, wobei das Benutzerschnittstellenmodul eine Schnittstelle auf einer Anzeige (25) des Glukosemessgerätes (12) zusammen mit der Übertragung der Glukosemessung zu der Diabetesmanagementanwendung (14) anzeigt, wobei die Schnittstelle einen Hinweis auf die Datenübertragung, einen Zahlenwert für die Glukosemessung und eine Identifikation für die tragbare Rechenvorrichtung (16) bereitstellt.

## Revendications

1. Procédé mis en oeuvre par ordinateur de transmission d'une mesure de glucose automatiquement d'un glucomètre manuel (12) à une application de gestion du diabète (14) résidant sur un dispositif informatique portable (16), comprenant :
- la détermination, par le glucomètre (12), d'une mesure de glucose dans le sang à partir d'une bandelette de test insérée dans un orifice (21) du glucomètre (12), la bandelette de test ayant un site de réaction pour recevoir un échantillon de sang d'un patient ;
- l'affichage, par le glucomètre (12), d'une interface de résultat de mesure sur un écran d'affichage (25) du glucomètre (12), où l'interface de résultat de mesure inclut une valeur numérique pour la mesure de glucose et est affichée en réponse à la détermination de la mesure de glucose dans le sang ; et
- la transmission, par le glucomètre (12), d'un message par l'intermédiaire d'une liaison de données sans fil à l'application de gestion du diabète (14) ;
**caractérisé en ce que**
- la transmission du message se produit automatiquement en réponse à la navigation loin de l'interface de résultat de mesure ; et
**en ce que** le procédé comprend en outre :
- le marquage, par le glucomètre (12), de la mesure de glucose avec un numéro de séquence unique, où le numéro de séquence est déterminé à partir d'un compteur résidant sur le glucomètre (12) ; et
- l'incrémentation, par le glucomètre (12), du compteur après l'étape de marquage de la mesure de glucose avec un numéro de séquence.

2. Procédé selon la revendication 1 comprenant en outre la transmission du message conformément à une caractéristique de basse énergie du standard de technologie sans fil Bluetooth.

3. Procédé selon la revendication 1 ou 2 comprenant en outre :
- la réception, par le glucomètre (12), d'une requête pour une mesure de glucose manquante, où la requête identifie la mesure de glucose manquante par un numéro de séquence attribué par le glucomètre (12) et la requête est envoyée par l'intermédiaire de la liaison de données sans fil par l'application de gestion du diabète (14) en réponse à la réception du message ; et
- la transmission, par le glucomètre (12), de la mesure de glucose manquante par l'intermédiaire de la liaison de données sans fil à l'application de gestion du diabète (14), où la transmission se produit automatiquement en réponse à la réception de la requête.

4. Procédé selon l'une des revendications précédentes qui comprend en outre l'affichage, par le glucomètre (12), d'une interface sur le dispositif d'affichage (25) du glucomètre (12) simultanément avec la transmission du message à l'application de gestion du diabète (14), où l'interface fournit une indication du transfert de données, une valeur numérique pour la mesure de glucose dans le sang et un identifiant pour le dispositif informatique portable (16).

5. Procédé selon l'une des revendications précédentes comprenant en outre :
- l'appariement, par le glucomètre (12), avec le dispositif informatique portable (16) pour établir ainsi la liaison de données sans fil ;
- l'invitation, par le glucomètre (12), à sélectionner un nom pour le dispositif informatique portable (16) ; et
- la réception, par le glucomètre (12), d'un nom pour le dispositif informatique portable (16) en réponse à l'invitation, où le nom du dispositif informatique portable (16) sert d'identifiant pour le dispositif informatique portable (16) sur l'interface.

6. Procédé selon l'une des revendications précédentes comprenant en outre :
- la réception, par le glucomètre (12), de l'heure actuelle du dispositif informatique portable (16) pendant la transmission du message ; et
- la synchronisation, par le glucomètre (12), d'une horloge maintenue par le glucomètre (12) avec l'heure actuelle reçue du dispositif informatique portable (16) lorsqu'une différence entre l'heure de l'horloge et l'heure actuelle dépasse un seuil de variance.

7. Procédé selon l'une des revendications précédentes qui comprend en outre la transmission du message automatiquement en réponse à la navigation de l'interface de résultat de mesure jusqu'à un écran de menu.

8. Procédé selon l'une des revendications précédentes comprenant en outre :
- la navigation, en réponse à une instruction d'entrée, de l'interface de résultat de mesure jusqu'à un écran de sélection de commentaire, l'écran de sélection de commentaire présentant une liste de commentaires pour la sélection ;
- la réception d'une sélection d'un commentaire de la liste de commentaires ;
- la navigation de l'écran de sélection de commentaire jusqu'à l'interface de résultat de mesure, la navigation en sens inverse jusqu'à l'interface de résultat de mesure en réponse à la réception de la sélection ; et
- le transfert de la mesure de glucose dans le sang en réponse à la navigation loin de l'interface de résultat de mesure.

9. Procédé selon l'une des revendications précédentes qui comprend en outre la récupération de la mesure de glucose manquante à partir d'un comptoir de données en utilisant le numéro de séquence de la requête, où la récupération est en réponse à la réception de la requête pour la mesure de glucose manquante.

10. Procédé selon l'une des revendications précédentes dans lequel le compteur est incrémenté d'un après l'étape de marquage de la mesure de glucose avec un numéro de séquence.

11. Glucomètre manuel (12) ayant une interface utilisateur pour l'affichage de l'état pendant la transmission d'une mesure de glucose dans le sang, comprenant :
- un orifice (21) configuré pour recevoir une bandelette de test ayant un site de réaction permettant de recevoir un échantillon de sang d'un patient ;
- un module de mesure de glucose (22) pouvant fonctionner en coopération avec une bandelette de test insérée dans l'orifice (21) pour mesurer le glucose dans un échantillon de sang résidant sur la bandelette de test ;
- un module d'interface utilisateur configuré pour recevoir la mesure de glucose du module de mesure de glucose (22), le module d'interface utilisateur fonctionne en outre pour afficher la mesure de glucose sur une interface de résultat de mesure du glucomètre (12) immédiatement après la détermination de la mesure de glucose par le module de mesure de glucose (22) ;
- un émetteur-récepteur sans fil (28) en communication de données avec le module d'interface utilisateur et pouvant fonctionner pour communiquer un message automatiquement par l'intermédiaire d'une liaison de données sans fil à une application de gestion du diabète (14) résidant sur un dispositif informatique portable (16) ;
**caractérisé en ce que** la transmission du message se produit automatiquement en réponse à la navigation loin de l'interface de résultat de mesure et le message inclut uniquement une mesure de glucose ; et
**en ce que** le module d'interface utilisateur est en outre configuré pour marquer la mesure de glucose avec un numéro de séquence unique à partir d'un compteur, et pour incrémenter le compteur après l'étape de marquage de la mesure de glucose avec un numéro de séquence.

12. Glucomètre manuel (12) selon la revendication 11 dans lequel le module d'interface utilisateur est configuré pour recevoir l'heure actuelle du dispositif informatique portable (16) pendant la transmission de la mesure de glucose dans le sang et fonctionne pour synchroniser une horloge maintenue par le glucomètre (12) avec l'heure actuelle reçue du dispositif informatique portable (16) lorsqu'une différence entre l'heure de l'horloge et l'heure actuelle dépasse un seuil de variance.

13. Glucomètre manuel (12) selon la revendication 11 ou 12 dans lequel le module d'interface utilisateur navigue, en réponse à une instruction d'entrée, de l'interface de résultat de mesure jusqu'à un écran de sélection de commentaire, l'écran de sélection de commentaire présente une liste de commentaires pour la sélection ; et navigue en sens inverse de l'écran de sélection de commentaire jusqu'à l'interface de résultat de mesure en réponse à la réception d'une sélection de commentaire ; dans lequel l'émetteur-récepteur sans fil (28) transfère la mesure de glucose dans le sang en réponse à la navigation loin de l'interface de résultat de mesure.

14. Glucomètre manuel (12) selon l'une quelconque des revendications 11 à 13 dans lequel le module d'interface utilisateur est configuré pour recevoir une requête pour une mesure de glucose manquante du dispositif informatique portable (16) et interagit avec l'émetteur-récepteur sans fil (28) pour transmettre la mesure de glucose manquante par l'intermédiaire de la liaison de données sans fil à l'application de gestion du diabète (14), où la requête identifie la mesure de glucose manquante par un numéro de séquence attribué par le glucomètre (12) et la transmission se produit automatiquement en réponse à la réception de la requête.

15. Glucomètre manuel (12) selon l'une quelconque des revendications 11 à 14 dans lequel le module d'interface utilisateur fonctionne pour afficher une interface sur un écran d'affichage (25) du glucomètre (12) simultanément avec le transfert de la mesure de glucose à l'application de gestion du diabète (14), où l'interface fournit une indication du transfert de données, une valeur numérique pour la mesure de glucose et un identifiant pour le dispositif informatique portable (16).
